Europäisches Patentamt

European Patent Office

Office européen des brevets

(11) Veröffentlichungsnummer: **0 303 025 B1**

(12) **EUROPÄISCHE PATENTSCHRIFT**

(45) Veröffentlichungstag der Patentschrift: **30.09.92**

(21) Anmeldenummer: **88109691.1**

(22) Anmeldetag: **16.06.88**

(51) Int. Cl.⁵: **A61K 9/70**

(54) **Verfahren zur Herstellung einer Darreichungs- und/oder Dosierungsform für Arzneimittelwirkstoffe.**

(30) Priorität: **14.08.87 DE 3727232**

(43) Veröffentlichungstag der Anmeldung:
**15.02.89 Patentblatt 89/07**

(45) Bekanntmachung des Hinweises auf die
Patenterteilung:
**30.09.92 Patentblatt 92/40**

(84) Benannte Vertragsstaaten:
**AT BE CH DE ES FR GB GR IT LI LU NL SE**

(56) Entgegenhaltungen:
**EP-A- 0 219 762**
**DE-A- 1 939 437**
**DE-A- 2 849 495**

(73) Patentinhaber: **LTS Lohmann Therapie-**
**Systeme GmbH & Co. KG**
**Irlicherstrasse 55**
**W-5450 Neuwied 12(DE)**

(72) Erfinder: **Anhäuser, Dieter**
**Rengsdorfer Str. 4**
**W-5451 Melsbach(DE)**
Erfinder: **Klein, Robert-Peter**
**Wikinger Str. 3**
**W-5450 Neuwied 11(DE)**

(74) Vertreter: **Neidl-Stippler, Cornelia, Dr.**
**Rauchstrasse 2**
**W-8000 München 80(DE)**

## Beschreibung

Die Erfindung betrifft ein Verfahren zur Herstellung einer Darreichungs- und/oder Dosierungsform für Arzneimittelwirkstoffe mittels eines Druckverfahrens.

Der Einsatz von Druckverfahren für die Herstellung von Arzneimitteln wurde bereits in der EP-OS 219 762 beschrieben, wo eine zweidimensionale Darreichungsform eines Arzneimittels, bestehend aus einer wasserlöslichen Trägerfolie und einer wirkstoffhaltigen Schicht hergestellt wird, indem die Beschichtungsmasse kontinuierlich mittels eines Walzenauftragsverfahrens aus der Drucktechnik auf mindestens eine Seite der Trägerfolie aufgebracht wird. Die mit diesen Verfahren erreichbare Gewichtskonstanz beträgt bei einer Bahngeschwindigkeit von mehreren 100 m/min ca ± 10%. Dabei erfolgt der Auftrag der Beschichtungsmasse mit Walzen mit spezieller Feingravur, wobei die eingravierten Rillen zur Laufrichtung der Trägerfolie vorzugsweise einen Winkel von 45 Grad bilden. Mit diesen Verfahren werden Schichten von bis zu 80 g/qm übertragen; es können nur ebene Flächen bedruckt werden.

Diese Walzenauftragsverfahren sind insofern unvorteilhaft, als sie nur für große Flächen geeignet sind und in ihrer Auftragsgenauigkeit zu wünschen übrig lassen; ferner führen sie z.B. bei der Herstellung von runden Arzneimittelformen zu Anfall größerer Mengen Abfall.

Für leicht verdampfende Materialien, teure Wirkstoffe oder kleine Herstellungsmengen eignen sich diese große Vorrichtungen benötigenden Verfahren nicht. Sie sind auch schwierig in bestehende Fertigungsverfahren einzubeziehen. Ferner ist es nachteilig, daß sie nur ebene Flächen gleichmäßig bedrucken; unebene Flächen sind diesem Verfahren nicht zugänglich.

Für die Herstellung von Arzneimitteldarreichungsformen mit hochwirksamen, ggf. flüchtigen Wirkstoffen und hohen Anforderungen an die konstanten Dosismengen sowie ggf. unebenen Substraten, auf die der Stoff aufgebracht werden sollte, wurden bisher bspw. Dosierpumpen, z.B. Kolbendosierpumpen, für die Wirkstoffe eingesetzt, von denen genau dosierte Mengen abgegeben werden. Derartige Dosierpumpen sind teuer in der Anschaffung und aufwendig in der Wartung; insbesondere, wenn sie für die Dosierung sehr kleiner Mengen ausgelegt sind.

Es ist demzufolge Aufgabe der Erfindung, ein neues Verfahren zur präzisen Material-Auftragung bei der Herstellung von Arzneimittel-Darreichungs- und/oder Dosierungformen zu schaffen.

Die Aufgabe wird erfindungsgemäß durch ein Verfahren gelöst, bei dem das Druckverfahren ein Tampondruckverfahren ist.

Tampondruckverfahren sind seit 1968 bekannt und eignen sich auch zum Bedrucken unebener Oberflächen, denen sich flexible, das Druckmedium übertragende Tampons anpassen. Ein Tampondrucker ist bspw. in der DE-OS 19 39 437 beschrieben. Dabei ist das zu druckende Bildelement in einem Klischee, der Druckform, vertieft eingeätzt. Das Druckmedium wird in dieses Klischee überführt, nach einem Rakelvorgang, der das im Klischee aufgenommene Druckmedium dosiert, wird von dem Tampon das im Klischee verbliebene Druckmedium aufgenommen und auf den zu bedruckenden Gegenstand übertragen. Eine Übersicht über Anwendungen und Eigenschaften des Tampondruckverfahrens findet sich im Prospekt der Fa. TAMPOPRINT GmbH, Daimlerstraße 27/1 in Korntal-Münchingen, auf den zur Vermeidung von Wiederholungen in vollem Umfang bezug genommen wird.

Tampondrucker sind wegen ihrem geringen Platzbedarf für die erfindungsgemäße Verwendung besonders geeignet, da sie sich problemlos in Fabrikationsstraßen integrieren und auch kapseln lassen, was bei der Verarbeitung hochwirksamer Arzneistoffe zum Schutz des Bedienungspersonals aber auch zur Verminderung des Verdampfens von flüchtigen Substanzen hilfreich ist; außerdem kann in gekapselten Systemen unter Schutzgas, wie Stickstoff, Argon od. dgl. gearbeitet werden, was bei sauerstoffempfindlichen Substanzen sinnvoll sein kann.

Bisher ist noch nicht versucht worden, Tampondrucker für die Abgabe präzise gesteuerter Mengen Druckmedium mit einem Flächengewicht von über etwa 30 g/qm einzusetzen; überraschenderweise hat es sich gezeigt, daß das Tampondruckverfahren eine genaue Dosierung von Flächengewichten bis zu 200 g/qm in einem Druckvorgang mit einer Varianz von ±2% und darunter ermöglicht.

Dabei ist es möglich, wie in der parallelen europäischen Anmeldung EP-A-0 306 636 "Tampondrucker mit erhöhter Druckmediumsabgabe" mit gleichem Anmeldetag der Anmelderin angegeben, durch Variation des Tamponmaterials (bei weicheren Tampons wird mehr Druckmedium übertragen) und der Tamponoberfläche (Vergrößerung durch Einbringen von Vertiefungen, wie Rillen, Näpfchen u.dgl.) sowie der Viskosität des Druckmediumsdie Menge Druckmedium, die in einem Druckvorgang abgegeben wird, in unerwarteter Weise zu erhöhen und trotzdem eine in engen Grenzen gleichbleibende Dosierung zu erhalten.

Durch das erfindungsgemäße Verfahren können nun erstmals kleine Wirkstoffdosen ohne Wirkstoffverlust, wie er bei den bisher verwendeten Flächenbeschichtungsverfahren bspw. für transdermale therapeutische Systeme der Fall war, in einer erwünschten Ausgestaltung, wie einem Kreis, Dreieck, Oval od. dgl. hergestellt werden, wodurch bei Auftragung von Wirkstoffen teurer Wirkstoff gespart und die problematische Wirkstoffabfallbeseitigung umgangen werden kann.

Bei einer bevorzugten Ausführungsform des erfindungsgemäßen Verfahrens ist die Darreichungs- und Dosierungsform ein dermal applizierbares System. Unter "dermaler Absorption" wird hier jegliche Absorption durch Haut, eingeschlossen Schleimhäute, verstanden, also auch rektale und vaginale Anwendungsformen, wie Zäpfchen od. dgl.

Besonders bevorzugte dermal applizierbare Systeme sind transdermale therapeutische Systeme, bspw. ein wirkstoffhaltiges Pflaster, wie sie bspw. in der DE-PS 36 29 304.0 beschrieben sind.

Das erfindungsgemäße Verfahren kann auch angewendet werden, wenn die Darreichungsform ein oral applizierbares System, wie eine Tablette, Kapsel od. dgl. ist.

Durch das erfindungsgemäße Verfahren können viele Bestandteile einer Darreichungsform hergestellt werden wie Klebeschichten und -punkte, Beschriftungen auf Tabletten sowie wirkstoffhaltige Bereiche u.s.w.

Es ist nun ferner möglich, in einem Herstellungsschritt durch Verwendung geeigneter Klischees mehrere Druckmedien gleichzeitig aufzutragen, so bspw. einen Klebstoffbereich und einen aseptischen oder wirkstoffhaltigen Bereich bei Pflastern oder therapeutischen Systemen. Das Klischee wie auch der Tampon selbst können temperiert werden, falls temperaturempfindliche oder auch nur in erwärmten Zustand verarbeitbare Materialien, wie bestimmte Klebstoffe, verarbeitet werden sollen.

Mit einem Druckvorgang kann ein Flächengewicht von bis zu etwa 200 g pro Quadratmeter an Druckmedium übertragen werden, eine weitere Verbesserung der Übertragungsmenge läßt sich durch dem Fachmann geläufige Verbesserungen der Verfahrens erreichen, wie sie insbesondere in der Parallelanmeldung der Anmelderin EP-A-0 306 636, betreffend eine "Tampondruckvorrichtung zur Übertragung definierter Mengen Druckmedium pro Flächeneinheit" beschrieben sind.

Ein besonderer Vorteil des erfindungsgemäßen Verfahrens liegt auch in der entscheidend verbesserten Arzneimittelsicherheit, denn hochwirksame Medikamente können weitaus genauer, als bisher möglich, dosiert werden. Der Auftrag überschüssigen Materials wird umgangen.

Weitere Merkmale und Vorteile der Erfindung werden nachstehend anhand der begleitenden Zeichnung erläutert. Dabei zeigt:

Fig.1      einen Tampondrucker bei der Auftragung einer Wirkstoffschicht auf ein pflasterartiges therapeutisches System;

Fig.2      den Tampondrucker der Fig.1 bei der Übernahme von neuem Wirkstoff aus dem Klischee;

Fig.3      den mit Druckmedium beschichteten Tampon bei der Übertragung vom Klischee auf das Substrat;

Fig.4      einen Querschnitt durch ein nach dem erfindungsgemäßen Verfahren hergestelltes transdermales pflasterartiges therapeutisches System; und

Fig.5      eine Draufsicht auf ein weiteres durch das Tampondruckverfahren hergestelltestransdermales therapeutischesSystem mit abgenommener Klebstoffschicht und mit zwei unterschiedlichen Wirkstoffen; sowie

Fig.6      eine weitere nach dem erfindungsgemäßeb Verfahren hergestellte bevorzugte Darreichungsform, eine Tablette.

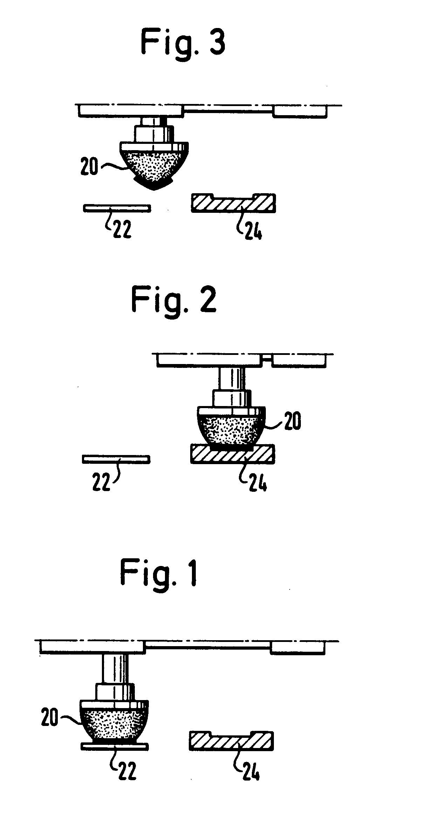

Wie in Fig. 1 dargestellt, wird ein elastischer eiförmiger Silikongummitampon 20 mit wirkstoffhaltiger Druckmediums-Beschichtung auf ein flaches Substrat, hier auf eine für eine Pflasterrückschicht geeignete Polyethylen-Folie 22 gedrückt, um nach seinem Hochfahren die Beschichtung auf dem Substrat 22 zu lassen.

In Fig. 2 ist gezeigt, wie die Aufnahme des wirkstoffhaltigen Druckmediums erfolgt. Der Tampon 20 wird auf ein Klischee 24 - eine mit einer Vertiefung, in der das Druckmedium eingebracht bevorzugt durch ein Rakelmesser (nicht gezeigt) geglättet und dosiert wird versehene Platte, gedrückt, um das in der Vertiefung befindlichen Druckmedium aufzunehmen. Anschließend schwenkt der Tampon 20, wie in Fig.3 gezeigt, druckmediums-beschichtet zum Substrat 22, um dieses zu bedrucken. Die Zufuhr des Druckmediums kann taktweise erfolgen, wobei die taktweise Nachlieferung durch einen mit dem Klischee 24 abschließenden Vorratsbehälter erfolgt, in dem sich das Druckmedium befindet. Dieser Vorratsbehälter wird taktmäßig, während der Tampon 20 auf das Substrat 22 druckt, über die Vetiefung im Klischee geschoben und läßt dort eine durch die Ätztiefe bestimmte gleichmäßig verteilte Druckmediumsmenge zurück, die nach Wegschwenken der Klischees und gleichzeitigem Abstreifen des überschüssigen Druckmediums im nächsten Takt vom Tampon 20 abgehoben und auf das nächste zu bedruckende Substrat 22 transferiert wird. Die bedruckte Substratschicht kann nun sogleich von einer Deckschicht in der nächsten Station der Fertigungsstraße überdeckt werden, so daß keine längere Aussetzung des Druckmediums gegenüber der Atmosphäre erfolgt, was zu Schäden der Substanz bei lichtempfindlichen Substanzen; zu Verdampfung bei Druckmedien mit hohem Dampfdruck u.dgl. führen könnte.

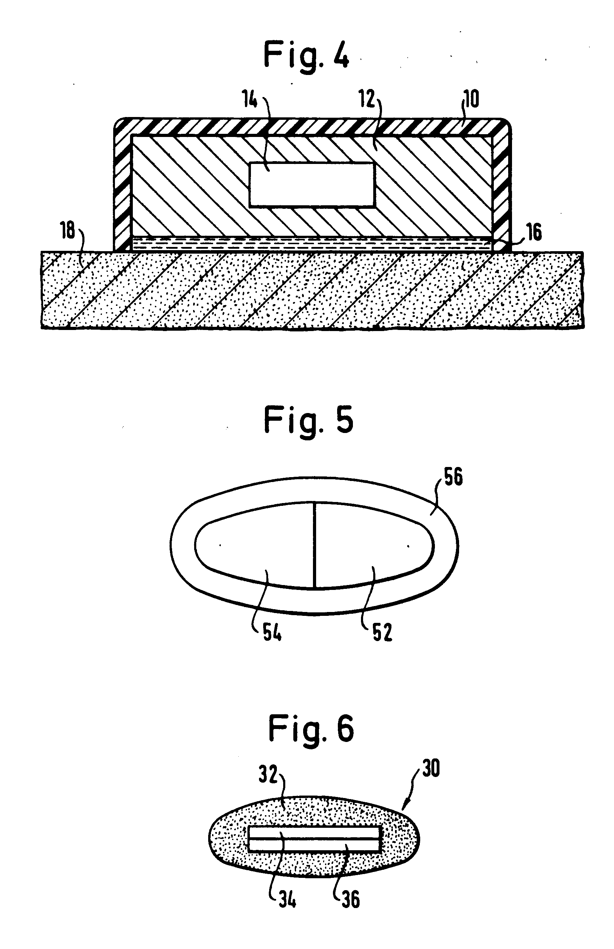

In Fig. 4 ist ein zumindest teilweise nach dem erfindungsgemäßen Verfahren hergestelltes pflasterartiges transdermales therapeutisches System dargestellt. Das System ist durch eine Deckfolie 10, die wirkstoffundurchlässig ist, abgedeckt. Unter der Deckfolie befindet sich eine durch das erfindungsgemäße Druckverfahren in mehreren Schritten auftragbare Wirkstoffverteilungsmatrix 12, die hier haftklebend ist. In die Haftklebeschicht ist ein Wirkstoffsreservoir 14 mit anderer Zusammensetzung eingebettet. Schließlich folgt eine durch das erfindungsgemäße Verfahren auftragbare Steuerschicht 16 mit vorherbestimmter Dicke, um die aus der Wirkstoffverteilungsmatrix herausdiffundierenden Wirkstoffe zu steuern.

In Fig. 5 ist eine weitere Auftragungsmöglichkeit mit dem erfindungsgemäßen Verfahren für transdermale Systeme dargestellt: dabei wurde die Rückschicht des Systems abgenommen und die beiden Wirkstoffreservoirs 52, 54 sind vollständig sichtbar.

Hier werden zwei halbovalförmige wirkstoffhaltige Schichten 52, 54 auf eine Klebematerialschicht 56 aufgedruckt, die anschließend von einer (hier abgenommenne und nicht gezeigten) klebenden Rückschicht überschichtet werden. Hier zeigt sich auch die überlegende Arbeitsweise des erfindungsgemäßen Verfahrens bei der Herstellung von Mehrkomponentensysteme mit hoher Beschichtungsgenauigkeit. In Fig. 6 ist eine Tablette 30 mit Zukkermantel 32 dargestellt, die ein zweischichtiges Depot aufweist. Der Zukkermantel umschließt ein zweischichtiges Wirkstoffpräparat, mit den Schichten 36, 34, von denen die Schicht 36 durch Aufdruken auf 34 mit dem Tampondruckverfahren hergestellt wurde.

Nachfolgend wird eine bevorzugte Variante des erfindungsgemüßen Verfahrens bei der Herstellung von Nicotinpflastern beschrieben.

Beispiel:

Herstellung eines Nicotinpflasters

Ein Tampondrucker mit einer Stahl-Klischeeplatte mit einer runden Vertiefung von 39 mm und einer Ätztiefe von 240 Micrometern als Druckbild wurde zum Bedrucken von Substraten eingesetzt. Als Tampon wurde ein eiförmiger Silikon-Schaumgummitampon mit einer Shore-Härte von 6 eingesetzt.

Als Druckmedium wurde eine 50 Gew.%-ige Lösung von Nicotin in Miglyol 812, einem von der Fa DYNAMIT NOBEL hergestellten und vertriebenen Öl, mit einer Viskosität von 44 dPa/s bei 21 Grad Celsius eingesetzt. Mittels des Tampons wurden kreisförmige Schichten dieses Druckmediums auf ein haftkleberbeschichtetes Substrat gesetzt. Als Substrat wurde eine aluminiumbedampfte 100 Micrometer dicke, silikonisierte Polyesterfolie- die spätere vor Gebrauch des Pflasters abziehbare Schutzschicht - mit einer Acrylat-Haftkleberschicht (30 g/qm) eingesetzt, dabei wurde auf die Acrylatoberfläche gedruckt. Es wurden Lösungsmengen von 91 mg auf einer Fläche von 12 cm$^2$ bei einer Fehlergrenze unter 5% aufgebracht.

Anschließend wurde das bedruckte Substrat in an sich bekannter Weise mit einer die Rückschicht des Pflasters bildenden 16 Micrometer dicken, aluminiumbedampften Polyesterfolie mit einem Acrylat-Grundierungsstrich kaschiert und konfektioniert.

Der Aufbau des fertigen Nicotinpflasters entspricht in etwa dem Aufbau des in Fig. 4 gezeigten Pflasters.

**Patentansprüche**

1. Verfahren zur Herstellung einer Darreichungs- und/oder Dosierungsform für Arzneimittelwirkstoffe unter Einsatz eines Druckverfahrens, dadurch gekennzeichnet, daß das Druckverfahren ein Tampondruckverfahren ist.

2. Verfahren nach Anspruch 1, dadurch gekennzeichnet, daß die Darreichungs- und Dosierungsform ein dermal applizierbares System ist.

3. Verfahren nach Anspruch 1, dadurch gekennzeichnet, daß die Darreichungsform ein oral applizierbares System ist.

4. Verfahren nach einem der Ansprüche 1 oder 2, dadurch gekennzeichnet, daß das dermal applizierbare System ein transdermales therapeutisches System ist.

5. Verfahren nach einem der vorangehenden Ansprüche, dadurch gekennzeichnet, daß mindestens ein Druckmedium wirkstoffhaltig ist.

6. Verfahren nach einem der Ansprüche 1-4, dadurch gekennzeichnet, daß mindestens ein Druckmedium wirkstofffrei ist.

7. Verfahren nach einem der vorangehenden Ansprüche, dadurch gekennzeichnet, daß ein zu bedruckendes Substrat eben oder uneben sein kann.

8. Verfahren nach einem der vorangehenden Ansprüche, dadurch gekennzeichnet, daß mit einem Druckvorgang bis zu etwa 200 g pro Quadratmeter an Druckmedium übertragen werden.

## Claims

1. Method for producing an administration and/or dosage form for pharmaceutical active substances using a printing method, characterised in that the printing method is a tampon printing method.

2. Method according to claim 1, characterised in that the administration and dosage form is a system which can be applied dermally.

3. Method according to claim 1, characterised in that the administration form is a system which can be applied orally.

4. Method according to either of claims 1 or 2, characterised in that the system which can be applied dermally is a transdermal therapeutic sytem.

5. Method according to one of the preceding claims, characterised in that at least one printing medium contains active substance.

6. Method according to one of claims 1 to 4, characterised in that at least one printing medium is free of active substance.

7. Method according to one of the preceding claims, characterised in that a substrate to be printed can be even or uneven.

8. Method according to one of the preceding claims, characterised in that in one printing procedure up to approximately 200 g per square metre of printing medium are transferred.

## Revendications

1. Procédé de fabrication d'une forme de présentation et/ou de dosage pour produits pharmaceutiques par mise en oeuvre d'un procédé d'impression, caractérisé en ce que le procédé d'impression est un procédé d'impression tampon.

2. Procédé selon le revendication 1, caractérisé en ce que la forme de présentation ou de dosage est un système applicable par voie dermique.

3. Procédé selon la revendication 1, caractérisé en ce que la forme de présentation est un système applicable par voie orale.

4. Procédé selon l'une quelconque des revendications 1 et 2, caractérisé en ce que le système applicable par voie dermique est un système applicable par voie dermique est un système thérapeutique transdermique.

5. Procédé selon l'une quelconque des revendications précédentes, caractérisé en ce qu'au moins un agent d'impression contient un principe actif.

6. Procédé selon l'une quelconque des revendications 1 à 4, caractérisé en ce qu'au moins un agent d'impression est exempt de principe actif.

7. Procédé selon l'une quelconque des revendications présédentes, caractérisé en ce que le substrat à imprimer peut être régulier ou inégal.

8. Procédé selon l'une quelconque des revendications précédentes, caractérisé en ce que l'on applique environ 200 $g/m^2$ d'agent d'impression à chaque opération d'impression.

# Fig. 3

20

22

24

# Fig. 2

20

22

24

# Fig. 1

20

22

24

## Fig. 4

## Fig. 5

## Fig. 6